# EUROPEAN PATENT SPECIFICATION

(11) **EP 0 509 752 B1**
(45) Date of publication and mention of the grant of the patent: **04.08.1999**
(21) Application number: 92303330.2
(22) Date of filing: 14.04.1992
(51) Int. Cl.: A61K 31/38, A61K 31/445, A61K 31/54, A61K 31/535

(54) **Ophthalmic compositions comprising combinations of a carbonic anhydrase inhibitor and a beta-adrenergic antagonist**
Ophthalmische Zusammensetzungen, die Kombinationen von einem Carboanhydrase-Inhibitor und einem Beta-adrenergischen Antagonist enthalten
Compositions ophtalmiques contenant des combinaisons d'un inhibiteur d'anhydrase carbonique et d'un antagoniste bêta-adrénergique

(30) Priority: 17.04.1991 US 686718; 13.02.1992 US 834913
(43) Date of publication of application: 21.10.1992
(73) Proprietor: Merck & Co., Inc., Rahway New Jersey 07065-0900 (US)
(72) Inventor: Baldwin, John J., Gwynedd Valley, PA 19437 (US)
(74) Representative: Thompson, John Dr.

(56) References cited:
- US-A- 4 863 922
- INTERNATIONAL OPHTHALMOLOGY, vol. 15, supplement, 1991, pages 11,12; F.P. GUNNING et al.: "Medical treatment of glaucoma: developments in research on topical carbonic anhydrase inhibitors"
- FORTSCHR. OPHTHALMOL., vol. 88, no. 6, 1991, pages 846-847; N. PFEIFFER et al.: "Additive Wirkung von Timolol und dem lokalen Karboanhydrasehemmer MK-417 (Sezolamid)"
- ARCH. OPHTHALMOL., vol. 108, no. 4, April 1990, pages 511-513; R.-F. WANG et al.: "The ocular hypotensive effect of the topical carbonic anhydrase inhibitor L-671,152 in glaucomatous monkeys"
- STN INTERNATIONAL, KARLSRUHE, FILE PHAR, AN=7469, PJB Publications Ltd; "Sezolamide"
- STN INTERNATIONAL, KARLSRUHE, FILE PHAR, AN=3610, PJB Publications Ltd; "Dorzolamide"
- Book no. 6TH ED, 1989, 'BECKER-SHAFFER'S DIAGNOSIS AND TREATMENT OF GLAUCOMA', C:V MOSBY , ST LOUIS.

## Description

### SUMMARY OF THE INVENTION

This invention relates to novel ophthalmic compositions comprising the topical carbonic anhydrase inhibitor
(*S,S*)-(-)-5,6-dihydro-4-ethylamino-6-methyl-4H-thieno[2,3-b]thiopyran-2-sulfonamide-7,7-dioxide, or
an ophthamologically acceptable salt thereof and the β-adrenergic antagonist
timolol, or an ophthalmologically acceptable salt thereof.

The invention is also concerned with the use of the novel ophthalmic compositions in the treatment of ocular hypertension.

More particularly, it relates to such ophthalmic combinations and their use in the treatment of ocular hypertension and glaucoma, wherein the β-adrenergic antagonist is 1-(tert-butylamino)-3-[(4-morpholino-1,2,5-thiadiazol-3-yl)oxy]-2-propanol, or an ophthalmologically acceptable salt thereof which name includes the (S)-(-)- and (R)-(+)- enantiomers and any mixtures thereof, including racemic material. The (S)-(-)-enantiomer is generally known as timolol.

### BACKGROUND OF THE INVENTION

Glaucoma is a degenerative disease of the eye wherein the intraocular pressure is too high to permit normal eye function. As a result, damage may occur to the optic nerve head and result in irreversible loss of visual function. If untreated, glaucoma may eventually lead to blindness. Ocular hypertension, i.e., the condition of elevated intraocular pressure without optic nerve head damage or characteristic glaucomatous visual field defects, is now believed by the majority of ophthalmologists to represent merely the earliest phase in the onset of glaucoma.

Many of the drugs formerly used to treat glaucoma proved not entirely satisfactory. The early methods of treatment of glaucoma employing pilocarpine produced undesirable local effects that made this drug, though valuable, unsatisfactory as a first line drug. More recently, clinicians have noted that many β-adrenergic antagonists are effective in reducing intraocular pressure. While many of these agents are effective for this purpose, there exist some patients with whom this treatment is not effective or not sufficiently effective. Many of these agents also have other characteristics, e.g., membrane stabilizing activity, that become more apparent with increased doses and render them unacceptable for chronic ocular use.

The β-adrenergic antagonist (S)-1-(tert-butylamino)-3-[(4-morpholino-1,2,5-thiadiazol-3-yl)-oxy]-2-propanol, timolol, was found to reduce intraocular pressure and to be devoid of many unwanted side effects associated with pilocarpine and, in addition, to possess advantages over many other β-adrenergic antagonists, e.g., to be devoid of local anesthetic properties, to have a long duration of activity, and to display minimal loss of effect with increased duration of dosing.

Although pilocarpine and β-adrenergic antagonists reduce intraocular pressure, none of these drugs manifests its action by inhibiting the enzyme carbonic anhydrase, and thus they do not take advantage of reducing the contribution to aqueous humor formation made by the carbonic anhydrase pathway.

Agents referred to as carbonic anhydrase inhibitors block or impede this inflow pathway by inhibiting the enzyme carbonic anhydrase. While such carbonic anhydrase inhibitors are now used to treat intraocular pressure by systemic routes, they thereby have the distinct disadvantage of inhibiting carbonic anhydrase throughout the entire body. Such a gross disruption of a basic enzyme system is justified only during an acute attack of alarmingly elevated intraocular pressure, or when no other agent is effective.

For several years, the desirability of directing the carbonic anhydrase inhibitor to only the desired ocular target tissue has been recognized. Because carbonic anhydrase inhibitors have a profound effect in altering basic physiological processes, the avoidance of a systemic route of administation serves to diminish, if not entirely eliminate, those side effects caused by inhibition of carbonic anhydrase such as metabolic acidosis, vomiting, numbness, tingling, general malaise and the like. Topically effective carbonic anhydrase inhibitors are disclosed in U.S. Patent Nos. 4,386,098; 4,416,890; 4,426,388; 4,668,697; and 4,863,922 and PCT Publication WO 91/15486. As yet, no topically effective carbonic anhydrase inhibitors are generally available for clinical use.

Thus, when a carbonic anhydrase inhibitor is combined with a β-adrenergic antagonist, there is experienced an effect that reduces the intraocular pressure below that obtained by either medicament individually.

The activity of carbonic anhydrase inhibitors currently under development wanes 6 to 8 hours post-dose, meaning that as single agents these carbonic anhydrase inhibitors must be administered at least three times a day to maintain the desired lowering of intraocular pressure. The combination of this invention maintains the desired lowering of intraocular pressure for a full twelve hours. Because of this increased duration of action, the combination disclosed herein is effective when administered only twice a day. Patient compliance is anticipated to be greater with twice a day administration than with three times a day administration.

The use of oral carbonic anhydrase inhibitors in combination with the topical β-adrenergic antagonist timolol and the resulting multiplicity of their effects is disclosed in Berson et al., American Journal of Ophthalmology 1981, 92, 788-791. However, the combination of an oral carbonic anhydrase inhibitor with a topical β-adrenergic antagonist presents two disadvantages. The first disadvantage is that the systemic use of a carbonic anhydrase inhibitor inhibits carbonic anhydrase throughout the body and exerts the same profound negative effects on basic metabolism whether it is used alone or in combination with a topical β-adrenergic antagonist. Secondly, there is poor patient compliance with simultaneous administration of both an oral and topical medicament.

The combination disclosed herein is effective either by co-administration of the medicaments in one solution or as a combined therapy achieved by prior administration of either the carbonic anhydrase inhibitor or the β-adrenergic antagonist followed by administration of the other solution. The use of a single solution containing both active medicaments is preferred.

The combination of this invention is suggested in U.S. Patent No. 4,863,922, but a precise formulation of the relative combination of medicaments to give effective reduction of intraocular pressure is neither taught nor disclosed therein.

There exists a patient population insufficiently responsive to available β-adrenergic antagonists who will benefit from the combination disclosed herein. Because of the combined effect of the β-adrenergic antagonist and the carbonic anhydrase inhibitor, these otherwise refractory patients can obtain a marked beneficial reduction in intraocular pressure from such a combination.

Furthermore, there exists a patient population who will benefit from a combination where the minimal dosage of one or both of the medicaments is employed, thus minimizing the possibility of the occurrence of undesirable effects of one or both of the medicaments which would be more likely to become apparent with chronic use at the higher dosage.

### DETAILED DESCRIPTION OF THE INVENTION

The present invention relates to the use of
(a) 0.05 to 5% (w/w) of (*S,S*)-(-)-5,6-dihydro-4-ethylamino-6-methyl-4H-thieno-[2,3-b]thiopyran-2-sulfonamide-7,7-dioxide, or an ophthalmologically acceptable salt thereof; and
(b) 0.01 to 1.0% (w/w) of (*S*)-(-)-1-(*tert*-butylamino)3[(4-morpholino-1,2,5-thiadiazol-3-yl)oxy]-2-propanol, or an ophthalmologically acceptable salt thereof;
   for the manufacture of a medicament for the treatment of ocular hypertension or glaucoma in a patient who is insufficiently responsive to β-adrenergic antagonists.

According to a further aspect of the present invention, there is provided an ophthalmic formulation for the treatment of ocular hypertension or glaucoma in a patient population the members of which are insufficiently responsive to β-adrenergic antagonists, which comprises:
(a) 0.05 to 5% (w/w) of (*S,S*)-(-)-5,6-dihydro-4-ethylamino-6-methyl-4H-thieno-[2,3-b]thiopyran-2-sulfonamide-7,7-dioxide, or an ophthalmologically acceptable salt thereof;
(b) 0.01 to 1.0% (w/w) of (*S*)-(-)-1-(*tert*-butylamino)-3-[(4 morpholino-1,2,5-thiadiazol-3-yl)oxy]2-propanol, or an ophthalmologically acceptable salt thereof; and
(c) an ophthalmologically acceptable carrier.

According to a yet further aspect of the present invention, there is provided a process for preparing an ophthalmic formulation which comprises adding:
(a) 0.05 to 5% (w/w) of (*S,S*)-(-)-5,6-dihydro-4-ethylamino-6-methyl-4H-thieno-[2,3-b]thiopyran-2-sulfonamide-7,7dioxide, or an ophthalmologically acceptable salt thereof; and
(b) 0.01 to 1.0% (w/w) of (*S*)-(-)-1-(*tert*-butylamino)-3[(4-morpholino-1,2,5-thiadiazol-3-yl)oxy]-2-propanol, or an ophthalmologically acceptable salt thereof;
   to an opthalmologically acceptable carrier, optionally containing non-toxic auxilliary substances.

An alternative process for obtaining an ophthalmic formulation in the form of a solution comprises:
(1) suspending or dissolving in water:
   (a) 0.05 to 5% (w/w) of (*S,S*)-(-)-5,6-dihydro-4-ethylamino-6-methyl-4H-thieno-[2,3-b]thiopyran-2-sulfonamide-7,7-dioxide or an ophthalmologically acceptable salt thereof; and
   (b) 0.01 to 1.0% (w/w) of (*S*)-(-)-1-(*tert*-butylamino)-3-[(4-morpholino-1,2,5-thiadiazol-3-yl)oxy]-2-propanol, or an ophthalmologically acceptable salt thereof; together with non-toxic auxilliary substances which may go with an ophthalmologically acceptable carrier; and
(2) adjusting the pH of the composition obtained to 5.0-6.0 by the addition of a suitable reagent.

The preferred β-adrenergic antagonist for use in the novel composition of this invention is timolol as its maleate salt.

The novel ophthalmic formulations of this invention comprise about 0.05 to 5% (w/w) of carbonic anhydrase inhibitor, usually about 0.5 to 3% (w/w) and about 0.01 to 1% (w/w) of β-adrenergic antagonist, preferably about 0.1 to 0.5% (w/w) to be administered on a 1 to 2 times a day schedule.

The novel method of this invention comprises the topical ocular administration of about 0.025 to 5 mg per day, preferably about 0.25 to 3 mg per day, of carbonic anhydrase inhibitor and about 0.005 to 1 mg per day, preferably about 0.05 to 0.5 mg per day, of β-adrenergic antagonist to each eye.

As a unit dosage, between 0.025 and 2.5 mg of the carbonic anhydrase inhibitor and 0.005 to 0.5 mg of the β-adrenergic antagonist are applied to the eye; preferably, 0.25 to 1.5 mg of the carbonic anhydrase inhibitor and 0.05 to 0.25 mg of the β-adrenergic antagonist.

Suitable subjects for the administration of the formulation of the present invention include primates, man and other animals, particularly man and domesticated animals such as cats and dogs.

For topical ocular administration the novel formulations of this invention may take the form of solutions, gels, ointments, suspensions or solid inserts, formulated so that a unit dosage comprises a therapeutically effective amount of each active component or some submultiple thereof.

Typical ophthalmologically acceptable carriers for the novel formulations are, for example, water, mixtures of water and water-miscible solvents such as lower alkanols or aralkanols, vegetable oils, polyalkylene glycols, petroleum based jelly, ethyl cellulose, ethyl oleate, carboxymethylcellulose, polyvinylpyrrolidone, isopropyl myristate and other conventionally employed acceptable carriers. The pharmaceutical preparation may also contain non-toxic auxiliary substances such as emulsifying, preserving, wetting agents, bodying agents and the like, as for example, polyethylene glycols 200, 300, 400 and 600, carbowaxes 1,000, 1,500, 4,000, 6,000 and 10,000, antibacterial components such as quaternary ammonium compounds, phenylmercuric salts known to have cold sterilizing properties and which are non-injurious in use, thimerosal, benzalkonium chloride, methyl and propyl paraben, benzyldodecinium bromide, benzyl alcohol, phenylethanol, buffering ingredients such as sodium chloride, sodium borate, sodium acetate, or gluconate buffers, and other conventional ingredients such as sorbitan monolaurate, triethanolamine, polyoxyethylene sorbitan monopalmitylate, dioctyl sodium sulfosuccinate, monothioglycerol, thiosorbitol, ethylenediamine tetraacetic acid, and the like. Additionally, suitable ophthalmic vehicles can be used as carrier media for the present purpose including conventional phosphate buffer vehicle systems, isotonic boric acid vehicles, isotonic sodium chloride vehicles, isotonic sodium borate vehicles and the like.

The formulation may also include a gum such as gellan gum at a concentration of 0.1% to 2% by weight so that the aqueous eyedrops gel on contact with the eye, thus providing the advantages of a solid ophthalmic insert as described in U.S. Patent 4,861,760.

The pharmaceutical preparation may also be in the form of a solid insert such as one which after dispensing the drug remains essentially intact as described in U.S. Patents 4,256,108; 4,160,452; and 4,265,874; or a bio-erodible insert that either is soluble in lacrimal fluids, or otherwise disintegrates as described in U.S. Patent 4,287,175 or EPO publication 0,077,261.

The following examples of ophthalmic formulations are given by way of illustration.

### EXAMPLE 1

| SOLUTION COMPOSITION | I | II | III |
|---|---|---|---|
| (S,S)-(-)-5,6-dihydro-4-ethylamino-6-methyl-4H-thieno-[2,3b] thiopyran-2-sulfonamide-7,7-dioxide monohydrochloride | 22.26 g | 22.26 g | 1.113 g |
| (S)-(-)-1-(tert-butylamino)-3-[(4-morpholino-1,2,5-thiadiazol-3-yl)oxy]-2-propanol maleate | 6.834 g | 1.367 g | 6.834 g |
| Sodium citrate.2H₂O | 2.940 g | 2.940 g | 2.940 g |
| Benzalkonium Chloride | 0.075 g | 0.075 g | 0.075 g |
| Hydroxyethylcellulose | 5.00 g | 5.00 g | 5.00 g |
| Sodium hydroxide q.s. | pH = 6.0 | pH = 6.0 | pH = 6.0 |
| Mannitol | 16.00 g | 21.00 g | 35.90 g |
| Water for injection q.s. ad. | 1000 g | 1000 g | 1000 g |

The active compounds, sodium citrate, benzalkonium chloride (in a 50% W/W solution), and mannitol are dissolved in approximately 400 mL water for injection in a tared and sterile vessel. The pH of the composition is adjusted to 6.0 by addition of 0.2 N sodium hydroxide solution, and water for injection is added until the weight of composition equals 750 g. The composition is sterilized by filtration, pushing the solution with a 2 bar pressure of 0.45 micron filtrated nitrogen. Then 250 g of a 2% hydroxyethylcellulose autoclaved solution is added and the obtained solution is homogenized by stirring with a magnetic stirring bar. The solution is aseptically subdivided into 3.5 mL aliquots and sealed.

### EXAMPLE 2

| SOLUTION COMPOSITION | I | II |
|---|---|---|
| (S,S)-(-)-5,6-dihydro-4-ethylamino-6-methyl-4H-thieno[2,3b]-thiopyran-2-sulfonamide-7,7-dioxide monohydrochloride | 2.0 mg | 0.2 mg |
| (S)-(-)-1-(tert-butylamino)-3-[(4-morpholino-1,2,5-thiadiazol-3-yl)oxy]-2 propanol maleate | 0.5 mg | 0.5 mg |
| GELRITE™ gellan gum | 6.0 mg | 6.0 mg |
| Monobasic sodium phosphate .2H₂O | Quantity sufficient to give final pH 5.5 - 6.0 | |
| Dibasic sodium phosphate .12H₂O | | |
| Benzyldodecinium bromide | 0.10 mg | 0.10 mg |
| Polysorbate 80 | 0.2 mg | 0.2 mg |
| Water for injection q.s. ad. | 1.0 mL | 1.0 mL |

The active compounds, GELRITE™ gellan gum, phosphate buffer salts, benzyldodecinium bromide and Polysorbate 80 are added to and suspended or dissolved in water. The pH of the composition is adjusted to 5.5-6.0 and diluted to volume. The composition is rendered sterile by ionizing radiation.

### EXAMPLE 3

### Study of (S,S)-(-)-5,6-dihydro-4-ethylamino-6-methyl-4H-thieno[2,3b]thiopyran-2-sulfonamide-7,7-dioxide (I) in combination with Timolol

Patients aged 40 or over, with either ocular hypertension or primary open angle glaucoma with an intraocular pressure (IOP) in one or both eyes of 22 mmHg or more at one time point each day while receiving timolol 0.5% twice a day (bid) alone were admitted to the study. Patients had been on timolol 0.5% bid, either alone or in combination for at least three weeks prior to study entry and had been on timolol 0.5% bid as their sole glaucoma therapy for at least two weeks prior to study admission.

Secondary glaucoma was an exclusion as was a history of glaucoma surgery or laser trabeculoplasty/gonioplasty. Patients for whom timolol was contraindicated by the datasheet were excluded and also excluded were those on a concurrent β-blocker, carbonic anhydrase inhibitor, or clonidine. Thirty-one patients entered the study.

### Procedure

1. All patients had their visual fields plotted by Goldmann Perimetry prior to study entry.
2. Patients were admitted for a 12 hour diurnal curve (i.e., IOP recorded at 08.00, 09.00, 10.00, 12.00, 14.00, 16.00, 18.00, 20.00 hours approximately, the 08.00 recording was immediately prior to instillation of the drops). All pressures were measured by the same observer using the same Goldmann applanation tonometer.
3. Following recording of the baseline Diurnal Curve on timolol 0.5% bid, all of the patients were instructed to add 1 drop of a solution to each eye at 8:10 pm and 8:10 am, ten minutes after adding timolol, for seven days. The solution given to 16 of the patients contained 2% Compound I; the solution given to the other 15 patients was a placebo solution.
4. On Day 2, the IOP of each patient was measured at 8 am and 9 am, and a 12 hour diurnal curve was recorded on Day 8.

preliminary IOP data follow:

### MEAN IOP PRESTUDY AND PERCENT CHANGE IN IOP ON DAY 8 FROM PRESTUDY

| COMPOUND I PLUS TIMOLOL GROUP | | |
|---|---|---|
| TIME | TIMOLOL BASELINE | TIMOLOL PLUS COMPOUND I |
| 8am | 27.4 | -16.8 % |
| 9am | 27.1 | -21.0 % |
| 10am | 25.4 | -18.9 % |
| noon | 25.6 | -17.3 % |
| 2pm | 24.5 | -18.6 % |
| 4pm | 25.2 | -17.0 % |
| 6pm | 25.7 | -18.2 % |
| 8pm | 24.4 | -13.2 % |

| PLACEBO PLUS TIMOLOL GROUP | | |
|---|---|---|
| TIME | TIMOLOL BASELINE | TIMOLOL PLUS PLACEBO |
| 8am | 26.9 | - 3.4 % |
| 9am | 24.2 | - 4.5 % |
| 10am | 23.3 | - 1.7 % |
| noon | 23.2 | + 0.2 % |
| 2pm | 21.6 | + 0.1 % |
| 4pm | 22.7 | - 0.1 % |
| 6pm | 23.1 | - 3.7 % |
| 8pm | 21.9 | + 6.6 % |

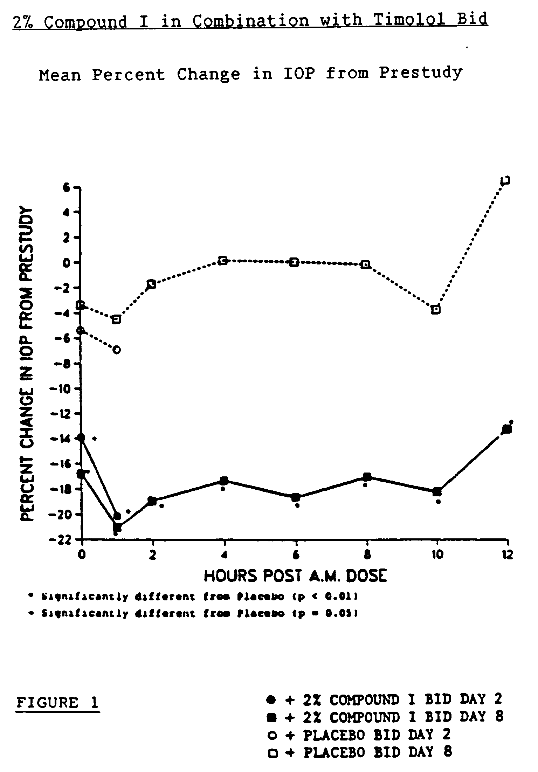

These data are represented graphically in Figure 1.

Overall, Compound I given every 12 hours demonstrated a clinically and statistically significant effect over the effect of timolol alone, ranging from 13%-21% based on worse eye analysis.

## Claims

1. Use of
(a) 0.05 to 5% (w/w) of (*S,S*)-(-)-5,6-dihydro-4-ethylamino-6-methyl-4H-thieno-[2,3-b]thiopyran-2-sulfonamide-7,7-dioxide, or an ophthalmologically acceptable salt thereof; and
(b) 0.01 to 1.0% (w/w) of (*S*)-(-)-1-(*tert*-butylamino)-3-[(4-morpholino-1,2,5-thiadiazol-3-yl)oxy]-2-propanol, or an ophthalmologically acceptable salt thereof;
for the manufacture of a medicament for the treatment of ocular hypertension or glaucoma in a patient who is insufficiently responsive to β-adrenergic antagonists.

2. Use as claimed in claim 1 wherein said ophthalmologically acceptable salt of (*S*)-(-)-1-(*tert*-butylamino)-3-[(4-morpholino-1,2,5-thiadiazol-3-yl)oxy]-2-propanol is the maleate salt.

3. Use as claimed in claim 1 or claim 2 wherein said ophthalmologically acceptable salt of (*S,S*)-(-)-5,6-dihydro-4-ethylamino-6-methyl-4H-thieno-[2,3-b]thiopyran-2-sulfonamide-7,7-dioxide is the monohydrochloride salt.

4. Use as claimed in any one of claims 1 to 3 wherein the concentration of (*S,S*)-(-)-5,6-dihydro-4-ethylamino-6-methyl-4H-thieno-[2,3-b]thiopyran-2-sulfonamide-7,7-dioxide is 0.5 to 3%.

5. Use as claimed in any one or claims 1 to 4 wherein the concentration of (*S*)-(-)-1-(*tert*-butylamino)-3[(4-morpholino-1,2,5-thiadiazol-3-yl)oxy]-2-propanol is 0.1 to 0.5%.

6. Use as claimed in any one of claims 1 to 5 wherein the concentration of (*S,S*)(-)-5,6-dihydro-4-ethylamino-6-methyl-4H-thieno-[2,3-b]thiopyran-2-sulfonamide-7,7-dioxide is 0.7 to 2.0% and the concentration of (*S*)-(-)-1-(*tert*-butylamino)-3-[(4-morpholino-1,2,5-thiadiazol-3-yl)oxy]-2-propanol is 0.5%.

7. Use as claimed in any one of claims 1 to 6, wherein said medicament further comprises 0.1% to 2% gellan gum.

8. Use as claimed in any one of claims 1 to 7 wherein said medicament takes the form of a solution, gel, ointment, suspension or solid insert.

9. Use as claimed in claim 8 wherein said medicament takes the form of a solution adapted for topical administration.

10. An ophthalmic formulation for the treatment of ocular hypertension or glaucoma in a patient population the members of which are insufficiently responsive to β-adrenergic antagonists, which comprises:
(a) 0.05 to 5% (w/w) of (*S,S*)-(-)-5,6-dihydro-4-ethylamino-6-methyl-4H-thieno-[2,3-b]thiopyran-2-sulfonamide-7,7-dioxide, or an ophthalmologically acceptable salt thereof;
(b) 0.01 to 1.0% (w/w) of (*S*)-(-)-1-(*tert*-butylamino)-3-[(4-morpholino-1,2,5-thiadiazol-3-yl)oxy]-2-propanol, or an ophthalmologically acceptable salt thereof; and
(c) an ophthalmologically acceptable carrier.

11. A formulation as claimed in claim 10 wherein the formulation comprises the maleate salt of (*S*)(-)-1-(*tert*-butylamino)-3-[(4-morpholino-1,2,5-thiadiazol-3-yl)oxy]-2-propanol.

12. A formulation as claimed in claim 10 or claim 11 wherein the formulation comprises the monohydrochloride salt of (*S,S*)-(-)-5,6-dihydro-4-ethylamino-6-methyl-4H-thieno-[2,3-b]thiopyran-2-sulfonamide-7,7-dioxide.

13. A formulation as claimed in any one of claims 10 to 12 wherein the concentration of (*S,S*)-(-)-5,6-dihydro-4-ethylamino-6-methyl-4H-thieno-[2,3-b]thiopyran-2-sulfonamide-7,7-dioxide is 0.5 to 3%.

14. A formulation as claimed in any one or claims 10 to 13 wherein the concentration of (*S*)-(-)-1-(*tert*-butylamino)-3-[(4-morpholino-1,2,5-thiadiazol-3-yl)oxy]-2-propanol is 0.1 to 0.5%.

15. A formulation as claimed in any one of claims 10 to 14 wherein the concentration of (*S,S*)-(-)-5,6-dihydro-4-ethylamino-6-methyl-4H-thieno-[2,3-b]thiopyran-2-sulfonamide-7,7-dioxide is 0.7 to 2.0% and the concentration of (*S*)-(-)1-(*tert*-butylamino)-3-[(4-morpholino-1,2,5-thiadiazol-3-yl)oxy]-2-propanol is 0.5%.

16. A formulation as claimed in any one of claims 10 to 15, further comprising 0.1% to 2% gellan gum.

17. A formulation as claimed in any one of claims 10 to 16 wherein the formulations obtained may take the form of solutions, gels, ointments, suspensions, or solid inserts.

18. A formulation as claimed in claim 17 wherein the formulation obtained takes the form of a solution.

19. A process for preparing an ophthalmic formulation as claimed in any one of claims 10 to 18, which comprises adding:
(a) 0.05 to 5% (w/w) of (*S,S*)-(-)-5,6-dihydro-4-ethylamino-6-methyl-4H-thieno-[2,3-b]thiopyran-2-sulfonamide-7,7-dioxide, or an ophthalmologically acceptable salt thereof; and
(b) 0.01 to 1.0% (w/w) of (*S*)-(-)-1-(*tert*-butylamino)-3-[(4-morpholino-1,2,5-thiadiazol-3-yl)oxy]-2-propanol, or an ophthalmologically acceptable salt thereof;
to an opthalmologically acceptable carrier, optionally containing non-toxic auxilliary substances.

20. A process as claimed in claim 19, for obtaining an ophthalmic formulation in the form of a solution, which comprises:
(1) suspending or dissolving in water:
(a) 0.05 to 5% (w/w) of (*S,S*)-(-)-5,6-dihydro-4-ethylamino-6-methyl-4H-thieno-[2,3-b]thiopyran-2-sulfonamide-7,7-dioxide or an ophthalmologically acceptable salt thereof; and
(b) 0.01 to 1.0% (w/w) of (*S*)-(-)-1-(*tert*-butylamino)-3-[(4-morpholino-1,2,5-thiadiazol-3-yl)oxy]-2-propanol, or an ophthalmologically acceptable salt thereof;
together with non-toxic auxilliary substances which may go with an ophthalmologically acceptable carrier; and
(2) adjusting the pH of the composition obtained to 5.0-6.0 by the addition of a suitable reagent.

## Patentansprüche

1. Die Verwendung von
(a) 0,05 bis 5% (Gew./Gew.) (S,S)-(-)-5,6-Dihydro-4-ethylamino-6-methyl-4H-thieno-[2,3-b]thiopyran-2-sulfonamid-7,7-dioxid oder eines ophthalmologisch annehmbaren Salzes davon und
(b) 0,01 bis 1,0% (Gew./Gew.) (S)-(-)-1-(tert.-Butylamino)-3-[(4/morpholino-1,2,5-thiadiazol-3-yl)oxy]-2-propanol oder eines ophthalmologisch annehmbaren Salzes davon
zur Herstellung eines Medikaments zur Behandlung von okulärer Hypertension oder Glaukom bei einem Patienten, der auf β-adrenergische Antagonisten unzureichend anspricht.

2. Die wie in Anspruch 1 beanspruchte Verwendung, bei der das ophthalmologisch annehmbare Salz von (S)-(-)-1-(tert.-Butylamino)-3-[(4-morpholino-1,2,5-thiadiazol-3-yl)oxy]-2-propanol das Maleatsalz ist.

3. Die wie in Anspruch 1 oder Anspruch 2 beanspruchte Verwendung, bei der das ophthalmologisch annehmbare Salz von (S,S)-(-)-5,6-Dihydro-4-ethylamino-6-methyl-4H-thieno-[2,3-b]thiopyran-2-sulfonamid-7,7-dioxid das Monohydrochloridsalz ist.

4. Die wie in irgendeinem der Ansprüche 1 bis 3 beanspruchte Verwendung, bei der die Konzentration von (S,S)-(-)-5,6-Dihydro-4-ethylamino-6-methyl-4H-thieno-[2,3-b]thiopyran-2-sulfonamid-7,7-dioxid 0,5 bis 3% beträgt.

5. Die wie in irgendeinem der Ansprüche 1 bis 4 beanspruchte Verwendung, bei der die Konzentration von (S)-(-)-1-(tert.-Butylamino)-3-[(4-morpholino-1,2,5-thiadiazol-3-yl)oxy]-2-propanol 0,1 bis 0,5% beträgt.

6. Die wie in irgendeinem der Ansprüche 1 bis 5 beanspruchte Verwendung, bei der die Konzentration von (S,S)-(-)-5,6-Dihydro-4-ethylamino-6-methyl-4H-thieno-[2,3-b]thiopyran-2-sulfonamid-7,7-dioxid 0,7 bis 2,0% und die Konzentration von (S)-(-)-1-(tert.-Butylamino)-3-[(4-morpholino-1,2,5-thiadiazol-3-yl)oxy]-2-propanol 0,5% beträgt.

7. Die wie in irgendeinem der Ansprüche 1 bis 6 beanspruchte Verwendung, bei der das Medikament ferner 0,1% bis 2% Gellangummi enthält.

8. Die wie in irgendeinem der Ansprüche 1 bis 7 beanspruchte Verwendung, bei der das Medikament die Form einer Lösung, eines Gels, einer Salbe oder einer Suspension einnimmt oder in fester Form eingeführt wird.

9. Die wie in Anspruch 8 beanspruchte Verwendung, bei der das Medikament die Form einer Lösung einnimmt, die zur topischen Verabreichung hergerichtet ist.

10. Eine ophthalmologische Formulierung zur Behandlung von okulärer Hypertension oder Glaukom bei einer Patientengruppe, deren Mitglieder auf β-adrenergische Antagonisten unzureichend ansprechen, die enthält:
(a) 0,05 bis 5% (Gew./Gew.) (S,S)-(-)-5,6-Dihydro-4-ethyl-amino-6-methyl-4H-thieno-[2,3-b]thiopyran-2-sulfonamid-7,7-dioxid oder eines ophthalmologisch annehmbaren Salzes davon,
(b) 0,01 bis 1,0% (Gew./Gew.) (S)(-)-1-(tert.-Butylamino)-3-[(4-morpholino-1,2,5-thiadiazol-3-yl)oxy]-2-propanol oder eines ophthalmologisch annehmbaren Salzes davon und
(c) einen ophthalmologisch annehmbaren Träger.

11. Eine wie in Anspruch 10 beanspruchte Formulierung, bei der die Formulierung das Maleatsalz von (S)-(-)-1-(tert.-Butylamino)-3-[(4-morpholino-1,2,5-thiadiazol-3-yl)oxy]-2-propanol enthält.

12. Eine wie in Anspruch 10 oder Anspruch 11 beanspruchte Formulierung, bei der die Formulierung das Monohydrochloridsalz von (S,S)-(-)-5,6-Dihydro-4-ethylamino-6-methyl-4H-thieno-[2,3-b]thiopyran-2-sulfonamid-7,7-dioxid enthält.

13. Eine wie in irgendeinem der Ansprüche 10 bis 12 beanspruchte Formulierung, bei der die Konzentration von (S,S)-(-)-5,6-Dihydro-4-ethylamino-6-methyl-4H-thieno-[2,3-b]thiopyran-2-sulfonamid-7,7-dioxid 0,5 bis 3% beträgt.

14. Eine wie in irgendeinem der Ansprüche 10 bis 13 beanspruchte Formulierung, bei der die Konzentration von (S)-(-)-1-(tert.-Butylamino)-3-[(4-morpholino-1,2,5-thiadiazol-3-yl)oxy]-2-propanol 0,1 bis 0,5% beträgt.

15. Eine wie in irgendeinem der Ansprüche 10 bis 14 beanspruchte Formulierung, bei der die Konzentration von (S,S)-(-)-5,6-Dihydro-4-ethylamino-6-methyl-4H-thieno-[2,3-b]thiopyran-2-sulfonamid-7,7-dioxid 0,7 bis 2,0% und die Konzentration von (S)-(-)-1-(tert.-Butylamino)-3-[(4-morpholino-1,2,5-thiadiazol-3-yl)oxy]-2-propanol 0,5% beträgt.

16. Eine wie in irgendeinem der Ansprüche 10 bis 15 beanspruchte Formulierung, die ferner 0,1% bis 2% Gellangummi enthält.

17. Eine wie in irgendeinem der Ansprüche 10 bis 16 beanspruchte Formulierung, bei der die erhaltenen Formulierungen die Form von Lösungen, Gelen, Salben oder Suspensionen einnehmen oder in fester Form eingeführt werden können.

18. Eine wie in Anspruch 17 beanspruchte Formulierung, bei der die erhaltene Formulierung die Form einer Lösung einnimmt.

19. Ein Verfahren zur Herstellung einer wie in irgendeinem der Ansprüche 10 bis 18 beanspruchten ophthalmologischen Formulierung, das die Zugabe von:
(a) 0,05 bis 5% (Gew./Gew.) (S,S)-(-)-5,6-Dihydro-4-ethyl-amino-6-methyl-4H-thieno-[2,3-b]thiopyran-2-sulfonamid-7,7-dioxid oder eines ophthalmologisch annehmbaren Salzes davon und
(b) 0,01 bis 1,0% (Gew./Gew.) (S)-(-)-1-(tert.-Butylamino)-3-[(4-morpholino-1,2,5-thiadiazol-3-yl)oxy]-2-propanol oder eines ophthalmologisch annehmbaren Salzes davon
zu einem ophthalmologisch annehmbaren Träger, der gegebenenfalls nichttoxische Hilfsstoffe enthält, umfaßt.

20. Ein wie in Anspruch 19 beanspruchtes Verfahren zur Herstellung einer ophthalmologischen Formulierung in der Form einer Lösung, das umfaßt:
(1) Suspendieren oder Auflösen in Wasser von:
(a) 0,05 bis 5% (Gew./Gew.) (S,S)-(-)-5,6-Dihydro-4-ethylamino-6-methyl-4H-thieno-[2,3-b]thiopyran-2-sulfonamid-7,7-dioxid oder eines ophthalmologisch annehmbaren Salzes davon und
(b) 0,01 bis 1,0% (Gew./Gew.) (S)-(-)-1-(tert.-Butylamino)-3-[(4-morpholino-1,2,5-thiadiazol-3-yl)oxy]-2-propanol oder eines ophthalmologisch annehmbaren Salzes davon
zusammen mit nichttoxischen Hilfsstoffen, die zusammen mit einem ophthalmologisch annehmbaren Träger verwendet werden können, und
(2) Einstellen des pH-Werts der erhaltenen Zusammensetzung auf 5,0-6,0 durch die Zugabe eines geeigneten Reagenzes.

## Revendications

1. Utilisation de
(a) de 0,05 à 5 % (p/p) de (S,S)-(-)-5,6-dihydro-4-éthylamino-6-méthyl-4H-thiéno-[2,3-b]thiopyran-2-sulfonamide-7,7-dioxyde, ou d'un de ses sels ophtalmologiquement acceptables ; et
(b) de 0,01 à 1,0 % (p/p) de (S)-(-)-1-(*tert*-butylamino)-3-[(4-morpholino-1,2,5-thiadiazol-3-yl)oxy]-2-propanol, ou d'un de ses sels ophtalmologiquement acceptables ;
pour la fabrication d'un médicament visant à traiter l'hypertension oculaire ou le glaucome chez un malade qui réagit insuffisamment aux antagonistes β-adrénergiques.

2. Utilisation selon la revendication 1, dans lequel ledit sel ophtalmologiquement acceptable du (S)-(-)-1-(*tert*-butylamino)-3-[(4-morpholino-1,2,5-thiadiazol-3-yl)oxy]-2-propanol est le sel de maléate.

3. Utilisation selon la revendication 1 ou la revendication 2 dans laquelle le sel ophtalmologiquement acceptable du (S,S)-(-)-5,6-dihydro-4-éthylamino-6-méthyl-4H-thiéno-[2,3-b]thiopyran-2-sulfonamide-7,7-dioxyde est le sel monochlorhydrate.

4. Utilisation selon l'une quelconque des revendications 1 à 3 dans laquelle la concentration de (S,S)-(-)-5,6-dihydro-4-éthylamino-6-méthyl-4H-thiéno-[2,3-b]thiopyran-2-sulfonamide-7,7-dioxyde est de 0,5 à 3 %.

5. Utilisation selon l'une quelconque des revendications 1 à 4 dans laquelle la concentration de (S)-(-)-1-(tert-butylamino)-3-[(4-morpholino-1,2,5-thiadiazol-3-yl)oxy]-2-propanol est de 0,1 à 0,5 %.

6. Utilisation selon l'une quelconque des revendications 1 à 5 dans laquelle la concentration de (S,S)-(-)-5,6-dihydro-4-éthylamino-6-méthyl-4H-thiéno-[2,3-b]thiopyran-2-sulfonamide-7,7-dioxyde est de 0,7 à 2,0 % et la concentration de (S)-(-)-1-(*tert*-butylamino)-3-[(4-morpholino-1,2,5-thiadiazol-3-yl)oxy]-2-propanol est de 0,5 %.

7. Utilisation selon l'une quelconque des revendications 1 à 6 dans laquelle ledit médicament comprend en outre de 0,1 % à 2 % de gomme de gellane.

8. Utilisation selon l'une quelconque des revendications 1 à 7, dans laquelle ledit médicament prend la forme d'une solution, d'un gel, d'un onguent, d'une suspension ou d'un insert solide.

9. Utilisation selon la revendication 6, dans laquelle ledit médicament prend la forme d'une solution adaptée pour l'administration locale.

10. Formulation ophtalmique pour le traitement de l'hypertension oculaire ou du glaucome dans une population de malades dont les membres réagissent insuffisamment aux antagonistes β-adrénergiques, qui comprend :
(a) de 0,05 à 5 % (p/p) de (S,S)-(-)-5,6-dihydro-4-éthylamino-6-méthyl-4H-thiéno-[2,3-b]thiopyran-2-sulfonamide-7,7-dioxyde, ou d'un de ses sels ophtalmologiquement acceptables ;
(b) de 0,01 à 1,0 % (p/p) de (S)-(-)-1-(tert-butylamino)-3-[(4-morpholino-1,2,5-thiadiazol-3-yl)oxy]-2-propanol, ou d'un de ses sels ophtalmologiquement acceptables ; et
(c) un support ophtalmologiquement acceptable.

11. Formulation selon la revendication 10 où la formulation comprend le sel de maléate du (S)-(-)-1-(tert-butylamino)-3-[(4-morpholino-1,2,5-thiadiazol-3- yl)oxy]-2-propanol.

12. Formulation selon la revendication 10 ou la revendication 11 où la formulation comprend le sel monochlorhydrate de (S,S)-(-)-5,6-dihydro-4-éthylamino-6-méthyl-4H-thiéno-[2,3-b]thiopyran-2-sulfonamide-7,7-dioxyde.

13. Formulation selon l'une quelconque des revendications 10 à 12 dans laquelle la concentration de (S,S)-(-)-5,6-dihydro-4-éthylamino-6-méthyl-4H-thiéno-[2,3-b]thiopyran-2-sulfonamide-7,7-dioxyde est de 0,5 à 3 %.

14. Formulation selon l'une quelconque des revendications 10 à 13 dans laquelle la concentration de (S)-(-)-1-(tert-butylamino)-3-[(4-morpholino-1,2,5-thiadiazol-3-yl)oxy]-2-propanol est de 0,1 à 0,5 %.

15. Formulation selon l'une quelconque des revendications 10 à 14 dans laquelle la concentration de (S,S)-(-)-5,6-dihydro-4-éthylamino-6-méthyl-4H-thiéno-[2,3-b]thiopyran-2-sulfonamide-7,7-dioxyde est de 0,7 à 2,0 % et la concentration de (S)-(-)-1-(tert-butylamino)-3-[(4-morpholino-1,2,5-thiadiazol-3-yl)oxy]-2-propanol est de 0,5 %.

16. Formulation selon l'une quelconque des revendications 10 à 15, comprenant en outre de 0,1 à 2 % de gomme de gellane.

17. Formulation selon l'une quelconque des revendications 10 à 16 où les formulations obtenues peuvent prendre la forme de solutions, de gels, d'onguents, de suspensions ou d'inserts solides.

18. Formulation selon la revendication 17, où la formulation obtenue prend la forme d'une solution.

19. Procédé de préparation d'une formulation ophtalmique telle que revendiquée dans l'une quelconque des revendications 10 à 18, dans lequel on ajoute
(a) de 0,05 à 5 % (p/p) de (S,S)-(-)-5,6-dihydro-4-éthylamino-6-méthyl-4H-thiéno-[2,3-b]thiopyran-2-sulfonamide-7,7-dioxyde, ou d'un de ses sels ophtalmologiquement acceptables ; et
(b) de 0,01 à 1,0 % (p/p) de (S)-(-)-1-(*tert*-butylamino)-3-[(4-morpholino-1,2,5-thiadiazol-3-yl)oxy]-2-propanol, ou d'un de ses sels ophtalmologiquement acceptables ;
à un support ophtalmologiquement acceptable, contenant facultativement des substances auxiliaires non toxiques.

20. Procédé selon la revendication 19, pour obtenir une formulation ophtalmique sous la forme d'une solution, dans lequel :
(1) on met en suspension ou on dissout dans l'eau:
(a) de 0,05 à 5 % (p/p) de (S,S)-(-)-5,6-dihydro-4-éthylamino-6-méthyl-4H-thiéno-[2,3-b]thiopyran-2-sulfonamide-7,7-dioxyde, ou d'un de ses sels ophtalmologiquement acceptables ; et
(b) de 0,01 à 1,0 % (p/p) de (S)-(-)-1-(*tert*-butylamino)-3-[(4-morpholino-1,2,5-thiadiazol-3-yl)oxy]-2-propanol, ou d'un de ses sels ophtalmologiquement acceptables ; avec des substances auxiliaires non toxiques qui peuvent être compatibles avec un support ophtalmologiquement acceptable ; et
(2) on ajuste le pH de la composition obtenue à 5,0-6,0 par addition d'un réactif approprié.
